# EUROPÄISCHE PATENTANMELDUNG

(11) **EP 1 052 242 A1**
(43) Veröffentlichungstag der Anmeldung: **15.11.2000**
(21) Anmeldenummer: 00110117.9
(22) Anmeldetag: 10.05.2000
(51) Int. Cl.: C07C 209/18, C07C 37/60

(54) **Verfahren zur Herstellung von Phenolen und aromatischen Aminen**

(30) Priorität: 11.05.1999 DE 19921783
(71) Anmelder: BASF AKTIENGESELLSCHAFT, 67056 Ludwigshafen (DE)
(72) Erfinder: Breuer, Klaus, Dr., 67122 Altrip (DE); Baier, Michael, Dr., 68161 Mannheim (DE); Müller, Ulrich, Dr., 67435 Neustadt/Weinstrasse (DE); Rieber, Norbert, Dr., 68259 Mannheim (DE)
(74) Vertreter: Isenbruck, Günter, Dr.

(57) **Zusammenfassung**

In einem Verfahren zur Herstellung von phenolischen Verbindungen durch Gasphasen-Hydroxylierung von aromatischen C₁₋₆-Alkylreste und /oder Halogenatome substituiert sein können, mit N₂O an einem Katalysator, bei dem als Katalysator eisenhaltige Zeolithe, in denen das Molverhältnis SiO₂: Fe₂O₃ kleiner oder gleich 20 ist, eingesetzt werden.

## Beschreibung

Die Erfindung betrifft Verfahren zur Herstellung von Phenolen und aromatischen Aminen, insbesondere zur Herstellung von Anilin aus Benzol über Phenol.

Phenol wird in der Regel durch Spaltung von Cumolhydroperoxid nach Hock erhalten. Es kann weiterhin aus Benzol über Benzolsulfonsäure oder über Chlorbenzol und nachfolgende Behandlung mit Alkali, aus Toluol über Benzoesäure und aus Cyclohexan über Cyclohexanon/Cyclohexanol hergestellt werden.

Zudem ist es bekannt, Phenol durch Hydroxylierung von Benzol mit N₂O in Gegenwart eines Katalysators herzustellen. Entsprechende Verfahren sind beispielsweise in EP-A-0 406 050 beschrieben.

Anilin kann, neben anderen großtechnischen Verfahren wie Reduktion von Nitrobenzol mit Eisenfeilspänen und Salzsäure oder mit katalytisch erzeugtem Wasserstoff, auch durch Ammonolyse von Phenol erhalten werden. Dabei wird Phenol mit Ammoniak in Gegenwart eines Katalysators umgesetzt. Ein entsprechendes Verfahren, das an silicium- und aluminiumhaltigen Katalysa-toren durchgeführt wird, ist z.B. in US 3,860,650 beschrieben.

Aufgabe der vorliegenden Erfindung ist die Bereitstellung von Katalysatoren und Verfahren zur Herstellung von Phenolen aus aromatischen Kohlenwasserstoffen, wobei die Phenole anschließend zu aromatischen Aminen weiter umgesetzt werden können.
Die Aufgabe wird erfindungsgemäß gelöst durch ein Verfahren zur Herstellung von phenolischen Verbindungen durch Gasphasen-Hydroxylierung von aromatischen C₆₋₁₄-Kohlenwasserstoffen, die zusätzlich durch 1 bis 3 C₁₋₆-Alkylreste und/oder Halogenatome substituiert sein können, mit N₂O an einem Katalysator, wobei als Katalysator eisenhaltige Zeolithe mit vorzugsweise Pentasil-Struktur, in denen das Molverhältnis SiO2: Fe₂O₃ kleiner oder gleich 20 ist, eingesetzt werden.

Dabei beträgt im Katalysator das Molverhältnis SiO2:Fe₂O₃ vorzugsweise 10 bis 20. Bevorzugt werden als Katalysator eisenhaltige bzw. damit dotierte Zeolithe vom Strukturtyp MFI, MEL, MOR, BEA, MWW, FER, MTW oder MH/MEL-Mischstrukturen eingesetzt. Besonders bevorzugt wird ein eisenhaltiger Zeolith vom Strukturtyp MFI, MEL oder einer MFI/MEL-Mischstruktur eingesetzt. Besonders bevorzugt werden als Zeolithe ZSM-5 und ZSM-11 oder Mischphasen davon eingesetzt. Besonders bevorzugt als Katalysator ist ein eisenhaltiger Zeolith des Typs ZBM-20. Die Herstellung dieses Katalysators kann wie in DE-A 28 31 611 beschrieben erfolgen z.B. unter Verwendung von Hexamethylendiamin. Die Katalysatoren zeichnen sich durch eine hohe Aktivität und hohe Regenerationsstabilität aus. Sie können aufgrund des hohen Eisenoxidanteils z.B. in einem Riser-Reaktor von dem Reaktoraustrag durch ein Magnetfeld abgetrennt werden.

Für eine allgemeine Beschreibung der Aromatenhydroxylierung mit N₂O kann beispielsweise auf Catalysis Today 41(1998), 365 - 385 und Applied Catalysis A: General, 98 (1993), 1 - 20 verwiesen werden.

Als aromatische Kohlenwasserstoffe werden allgemein aromatische C₆₋₁₄-Kohlenwasserstoffe eingesetzt, die zusätzlich durch 1 bis 3 C₁₋₆-Alkylreste und/oder Halogenatome wie Chlor- oder Fluoratome substituiert sein können. Bei den aromatischen Kohlenwasserstoff-Grundgerüsten handelt es sich dabei um Benzol, Naphthalin, Anthracen oder Phenanthren. Sie sind vorzugsweise durch einen oder zwei C₁₋₆-, vorzugsweise C₁₋₃-Alkylreste und/oder Halogenatome wie Chlor- oder Fluoratome substituiert. Vorzugsweise liegen keine Substituenten oder ein oder zwei Methyl- oder Ethylreste vor. Beispiele sind Benzol, Monohalogenbenzole, Toluol, Pseudocumol, Anisol und 1,4-Dichlorbenzol. Besonders bevorzugt wird Benzol eingesetzt, das nach der Umsetzung in der Stufe (1) Phenol und in der Stufe (2) Anilin ergibt.

Die Umsetzung mit N₂O erfolgt vorzugsweise in einem Molverhältnis von Kohlenwasserstoff zu N₂O von 1: 100 bis 100: 1, bevorzugt von 1: 20 bis 50: 1, besonders bevorzugt 1: 1 bis 15 : 1 . Die Temperatur bei der Umsetzung beträgt vorzugsweise 250 bis 500°C, besonders bevorzugt 350 bis 450°C. Der Druck bei der Umsetzung beträgt vorzugsweise 0 bis 30 bar, besonders bevorzugt 0 bis 10 bar. Bei der Umsetzung von Benzol wird insbesondere bei einer Temperatur von 330 bis 450°C und einem Druck von 0 bis 10 bar gearbeitet. Neben den Kohlenwasserstoffen und N₂O können noch inerte Trägergase wie Stickstoff in die Umsetzung geführt werden. Dabei kann das Volumenverhältnis von Trägergas zu N₂O 0 bis 50, vorzugsweise 1 bis 20 betragen.

Die Erfindung betrifft auch die Verwendung von eisenhaltigen Zeolithen mit vorzugsweise Pentasil-Struktur, in denen das Molverhältnis SiO₂:Fe₂O₃ kleiner oder gleich 20 ist, als Katalysatoren bei der Hydroxilierung von aromatischen Kohlenwasserstoffen mit N₂O. Auch hier gelten die vorstehend bevorzugten Ausführungsformen.

Die nach dem erfindungsgemäßen Verfahren erhaltenen hydroxilierten aromatischen Kohlenwasserstoffe können weiter zu aromatischen Aminen umgesetzt werden.

Die Erfindung betrifft auch ein Verfahren zur Herstellung von aromatischen Aminen durch
(1) Gasphasen-Hydroxylierung von aromatischen C₆₋₁₄-Kohlenwasserstoffen, die zusätzlich durch 1-3 C₁₋₆-Alkylreste und/oder Halogenatome substituiert sein können, nach einem wie vorstehend beschriebenen Verfahren, und nachfolgend
(2) Gasphasen-Aminierung des hydroxylierten Produktes aus Stufe (1) mit Ammoniak.

Dabei wird die Umsetzung in Stufe 2 vorzugsweise in Gegenwart eines silicium- und aluminiumhaltigen, vorzugsweise zeolithischen Katalysators durchgeführt, der zusätzlich noch SiO₂, Al₂O₃, B₂O₃, P₂O₅, V₂O₅, WO₆, MoO₆ oder Mischungen davon enthalten kann. Bevorzugte Katalysatoren sind in US 3,860,650 beschrieben.

Bei der Gasphasen-Aminierung in Stufe (2) wird vorzugsweise mit einem Molverhältnis von hydroxyliertem Produkt aus Stufe (1) zu Ammoniak von 1 : 1 bis 1 : 100, besonders bevorzugt 1 : 3 bis 1 : 20 gearbeitet. Auch in dieser Stufe kann ein inertes Trägergas wie Stickstoff mitverwendet werden. Das Volumenverhältnis von Trägergas zu Ammoniak beträgt dann bis 50 : 1, besonders bevorzugt 1 : 1 bis 20 : 1.

Sämtliche Reaktionsteilnehmer, das heißt aromatischer Kohlenwasserstoff, N₂O und Ammoniak können bereits zu Beginn in die Stufe (1) geführt werden. Dabei können die Stufen (1) und (2) gleichzeitig durchgeführt werden, was jedoch zu erniedrigten Ausbeuten führen kann. Vorzugsweise wird der aromatische Kohlenwasserstoff erst nach der in Stufe (1) erfolgten Hydroxylierung mit Ammoniak versetzt und in Stufe (2) aminiert. Dazu wird die Umsetzung in der Regel in zwei getrennten Reaktoren durchgeführt, wobei Ammoniak mit einer Zwischeneinspeisung in den Gasstrom von Stufe (1) zu Stufe (2) eingespeist wird.

Die Stufen (1) und (2) können im selben Reaktor durchgeführt werden, wie dies insbesondere bei der gleichzeitigen Umsetzung der Fall ist. Es ist jedoch auch möglich, den Reaktoraustrag aus Stufe (1) zwischenzukondensieren und sodann im gleichen Reaktor unter Zusatz von Ammoniak weiter umzusetzen.

Bevorzugt wird die Hydroxylierung der Aromaten mit N₂O und die Aminierung zu dem aromatischen Amin in separaten Reaktoren durchgeführt, wobei der Reaktoraustrag aus der Hydroxylierung mit Ammoniak versetzt und anschließend in den Aminierungsreaktor überführt wird. Der Reaktionsaustrag kann dabei in einer zwischengeschalteten Destillation von überschüssigem bzw. restlichem Ausgangsstoff, zum Beispiel Benzol, befreit und destillativ gereinigt oder isoliert werden. Bei einer Hydroxylierung und Aminierung in einem Reaktor ist das Katalysatorbett sowohl mit dem Hydroxylierungskatalysator als auch mit dem Aminierungskatalysator bestückt. Diese Katalysatoren können auch identisch sein, d. h. im Reaktor befindet sich nur eine Aktivmasse. Das erfindungsgemäße Verfahren wird vorzugsweise kontinuierlich durchgeführt. Dabei wird die Stufe (1) vorzugsweise mit einem Umsatz von 5 bis 50 % bezüglich Ausgangsstoff (Aromat) bzw. 50 bis 100 % bezüglich N₂O, geführt. Aus sicherheitstechnischen Gründen ist in Stufe (1) eine Erzielung eines hohen Umsatzes bezüglich N₂O vorteilhaft, weil in diesem Fall bei Zusatz von Ammoniak vor der Stufe (2) keine prinzipiell explosionsfähigen Mischungen aus N₂O und Ammoniak entstehen können.

Die Umsetzung in den Stufen (1) und (2) kann an unterschiedlichen Katalysatoren oder gleichen eisendotierten zeolithischen Katalysatoren durchgeführt werden.

Nach Durchführung des erfindungsgemäßen Verfahrens der Stufe (1) können die eingesetzten Katalysatoren zyklisch regeneriert werden. Dabei erfolgt die Regeneration vorzugsweise durch Abbrennen unter Zusatz von Sauerstoff oder Sauerstoff liefernden Substanzen bei Temperaturen von Raumtemperatur (25°C) bis 750°C, durch Waschen mit geeigneten Lösungsmitteln, durch Waschen mit oxidierenden Mineralsäuren oder durch Waschen mit einer Wasserstoffperoxidlösung. Es kann auch mit N₂O regeneriert werden, wenn im Feed der Strom organischer Substanzen geschlossen und der Anteil an N₂O erhöht wird. Hierbei kann die Reaktortemperatur (unverändert) konstant gehalten werden. Die Regeneration kann je nach Regenerationsweise in flüssiger, gasförmiger oder überkritischer Fahrweise erfolgen.

Dabei kann während des regenerativen Abbrennens eines verkokten Hydroxylierungskatalysators eine Verminderung der CO-Entwicklung durch Edelmetalldotierung erreicht werden, wie es beispielsweise in WO 98/05616 beschrieben ist. Zudem kann die Standzeit des zeolithischen Katalysators durch Hydrothermalbehandlung und gegebenenfalls nachfolgende Säurewäsche verbessert werden, wie es in WO 98/07513 und WO 95/27560 bzw. US 5,672,777 beschrieben ist.

Die Standzeit eines Fe-dotierten MFI-Zeolithen kann auch durch Wäsche mit einer Dithionit-Lösung verbessert werden, wie es in WO 98/07516 beschrieben ist.

Durch Beimischen von Inertgasen zum Reaktorfeed kann die Exothermie der Reaktion vermindert werden und die Brennbarkeit des Reaktorfeeds oder des Reaktoraustrags vermindert werden. Ein derartiges Verfahren ist in WO 98/15514 beschrieben.

Die Erfindung wird durch die nachfolgenden Beispiele näher erläutert.

Der in den Beispielen eingesetzte Katalysator ZBM-20 wurde gemäß DE-A 28 31 611 hergestellt.

### Hydroxilierung

### Beispiel 1

24 Nl/h N₂, 1,5 Nl/h N₂O und 0,3 ml/min Benzol wurden bei 350°C in einen Rohrreaktor, der 10 g eines ZBM-20 enthielt, hineingefahren. Der Austrag wurde kondensiert und mittels Gaschromatographie (GC) untersucht. Ausbeute an Phenol 18%, Selektivität 99 %.

### Beispiel 2

24 Nl/h N₂, 1,5 Nl/h N₂O und 0,3 ml/min Toluol wurden bei 350°C in einen Rohrreaktor, der 10 g eines ZBM-20 enthielt, hineingefahren. Der Austrag wurde kondensiert und mittels GC untersucht. Ausbeute an Methylphenol (Kresol) 6%, Selektivität 31%.

### Beispiel 3

24 Nl/h N₂, 1,5 Nl/h N₂O und 0,3 ml/min Fluorbenzol wurden bei 350°C in einen Rohrreaktor, der 10 g eines ZBM-20 enthielt, hineingefahren. Der Austrag wurde kondensiert und mittels GC untersucht. Ausbeute an Fluorphenolen 13%, Selektivität 67%.

### Beispiel 4

24 Nl/h N₂, 1,5 Nl/h N₂O und 0,3 ml/min Benzol wurden bei 400°C in einen Rohrreaktor, der 10 g eines ZBM-20 enthielt, hineingefahren. Der Austrag wurde kondensiert und mittels GC untersucht. Ausbeute an Phenol 27%, Selektivität 96%.

### Hydroxilierung und Aminierung

### Beispiel 5

24 Nl/h N₂, 1,5 Nl/h N₂O und 0,3 ml/min Benzol wurden in einen Rohrreaktor, der 10 g eines ZBM-20 enthielt, hineingefahren. Der Austrag wurde kondensiert und anschließend ohne weitere Aufarbeitung mit einer Rate von 1,5 ml/min zusammen mit 24 Nl/h N₂ und 5 Nl/h Ammoniak in einen Rohrreaktor, der ebenfalls 10 g des ZBM-20 enthielt, hineingefahren. Dabei wurde Anilin in einer Ausbeute von 1 % bezüglich Benzol erhalten.

### Beispiel 6

1,5 Nl/h N₂O und 1,5 ml/min Benzol wurden in einen Reaktor, der 10 g eines ZBM-20 enthielt, hineingefahren. Der gasförmige Austrag wurde anschließend ohne weitere Aufarbeitung über eine beheizte Leitung zusammen mit 10 Nl/h Ammoniak in einen Rohrreaktor, der ebenfalls 10 g des Katalysators enthielt, hineingefahren. Dabei wurde Anilin in einer Ausbeute von 10 % bezüglich N₂O erhalten.

### Beispiel 7

24 Nl/h N₂, 1,5 Nl/h N₂O, 1,5 Nl/h NH₃ und 0,3 ml/min Benzol wurden in einen Rohrreaktor, der 10 g eines ZBM-20 enthielt, hineingefahren. Der Austrag wurde kondensiert. Dabei wurde Anilin in einer Ausbeute von 0,1 % bezüglich Benzol erhalten.

### Beispiel 8

24 Nl/h N₂, 1,5 Nl/h N₂O, 0,5 Nl/h NH₃ und 0,3 ml/min Benzol wurden in einen Rohrreaktor, der 10 g eines ZBM-20 enthielt, hineingefahren. Der Austrag wurde kondensiert. Dabei wurde Anilin in einer Ausbeute von 0,05 % bezüglich Benzol erhalten.

### Beispiel 9

24 Nl/h N₂, 1,5 Nl/h N₂O, 2,5 Nl/h NH₃ und 0,3 ml/min Benzol wurden in einen Rohrreaktor, der 10 g eines ZBM-20 enthielt, hineingefahren. Der Austrag wurde kondensiert. Dabei wurde Anilin in einer Ausbeute von 0,2 % bezüglich Benzol erhalten.

In den Beispielen 6 bis 9 wurde bei p = 1 bar (abs) und T = 350°C, in Reaktor 2 in Beispiel 6 jedoch 450°C gearbeitet.

## Patentansprüche

1. Verfahren zur Herstellung von phenolischen Verbindungen durch Gasphasen-Hydroxylierung von aromatischen C₆₋₁₄-Kohlenwasserstoffen, die zusätzlich durch 1 bis 3 C₁₋₆-Alkylreste und/oder Halogenatome substituiert sein können, mit N₂O an einem Katalysator, dadurch gekennzeichnet, daß als Katalysator eisenhaltige Zeolithe, in denen das Molverhältnis SiO₂: Fe₂O₃ kleiner oder gleich 20 ist, eingesetzt werden.

2. Verfahren nach Anspruch 1, dadurch gekennnzeichnet, daß im Katalysator das Molverhältnis SiO₂: Fe₂O₃ 10 bis 20 beträgt.

3. Verfahren nach Anspruch 1 oder 2, dadurch gekennnzeichnet, daß als Katalysator eisenhaltige Zeolithe vom Strukturtyp MFI, MEL, MOR, BEA, MWW, FER, MTW oder MFI/MEL-Mischstrukturen eingesetzt werden.

4. Verfahren nach Anspruch 3, dadurch gekennnzeichnet, daß als Katalysator ein eisenhaltiger Zeolith des Typs ZBM 20 eingesetzt wird.

5. Verfahren nach Anspruch 4, dadurch gekennzeichnet, daß der Zeolith unter Verwendung von Hexamethylendiamin hergestellt wurde.

6. Verwendung von eisenhaltigen Zeolithen, in denen das Molverhältnis SiO₂: Fe₂O₃ kleiner oder gleich 20 ist, als Katalysatoren bei der Hydroxylierung von aromatischen Kohlenwasserstoffen mit N₂O.

7. Verfahren zur Herstellung von aromatischen Aminen durch
(1) Gasphasen-Hydroxylierung von aromatischen C₆₋₁₄-Kohlenwasserstoffen, die zusätzlich durch 1-3 C₁₋₆-Alkylreste und/oder Halogenatome substituiert sein können, nach einem Verfahren gemäß einem der Ansprüche 1 bis 5, und nachfolgend
(2) Gasphasen-Aminierung des hydroxylierten Produktes aus Stufe (1) mit Ammoniak.

8. Verfahren nach Anspruch 7, dadurch gekennzeichnet, daß in Stufe (2) ein zeolithischer Katalysator eingesetzt wird, der zusätzlich noch SiO₂, Al₂O₃, TiO₂, ZrO₂, B₂O₃, P₂O₅, V₂O₅, WO₆, MoO₆ oder Mischungen davon enthalten kann.
